# FASCICULE DE BREVET EUROPEEN

(11) **EP 2 434 953 B1**
(45) Date de publication et mention de la délivrance du brevet: **04.05.2016**
(21) Numéro de dépôt: 10728818.5
(22) Date de dépôt: 25.05.2010
(51) Int. Cl.: A61B 5/16

(54) **SYSTEME DE DETECTION IN VIVO D'UNE ZONE FONCTIONNELLE DU CERVEAU HUMAIN ENTRAINANT EN CAS DE LESION UN SYNDROME DE NEGLIGENCE SPATIALE UNILATERALE**
SYSTEM FÜR DEN IN-VIVO-NACHWEIS EINES FUNKTIONSBEREICHS DES MENSCHLICHEN GEHIRNS ALS URSACHE EINER HALBSEITIGEN VERNACHLÄSSIGUNG IM FALLE EINER LÄSION
SYSTEM FOR THE IN VIVO DETECTION OF A FUNCTIONAL AREA OF THE HUMAN BRAIN THAT CAUSES HEMISPATIAL NEGLECT IN THE EVENT OF A LESION

(30) Priorité: 26.05.2009 FR 0953463
(43) Date de publication de la demande: 04.04.2012
(73) Titulaire: E(YE)Brain, 94200 Ivry sur Seine (FR)
(72) Inventeur: OLIVIERO, Bastien, 94300 Vincennes (FR); THIEBAUT DE SCHOTTEN, Michel, 75006 Paris (FR); BARTOLOMEO, Paolo, 75006 Paris (FR); KINKINGNEHUN, Serge, 94400 Vitry sur Seine (FR)
(74) Mandataire: Tanty, François
(86) Numéro de dépôt international: PCT/FR2010/051008
(87) Numéro de publication internationale: WO 2010/136718

(56) Documents cités:
- WO-A-2007/010242
- HUGUES DUFFAU: "New concepts in surgery of WHO grade II gliomas: functional brain mapping, connectionism and plasticity - a review" JOURNAL OF NEURO-ONCOLOGY, KLUWER ACADEMIC PUBLISHERS, BO, vol. 79, no. 1, 11 avril 2006 (2006-04-11) , pages 77-115, XP019404489 ISSN: 1573-7373
- DE SCHOTTEN MICHEL THIEBAUT ET AL: "Direct evidence for a parietal-frontal pathway subserving spatial awareness in humans" SCIENCE (WASHINGTON D C), vol. 309, no. 5744, 30 septembre 2005 (2005-09-30), pages 2226-2228, XP007910878 ISSN: 0036-8075
- RABUFFETTI M ET AL: "TOUCH-SCREEN SYSTEM FOR ASSESSING VISUO-MOTOR EXPLORATORY SKILLS IN NEUROPSYCHOLOGICAL DISORDERS OF SPATIAL COGNITION" MEDICAL AND BIOLOGICAL ENGINEERING AND COMPUTING, SPRINGER, HEILDELBERG, DE, vol. 40, no. 6, 1 novembre 2002 (2002-11-01), pages 675-686, XP001046459 ISSN: 0140-0118

## Description

L'invention concerne un système détection in vivo d'une zone fonctionnelle du cerveau humain entrainant en cas de lésion un syndrome de négligence spatiale unilatérale. Elle concerne également un procédé de cartographie cérébrale d'une telle zone.

Le domaine de l'invention est le domaine de :
- la détection de zone fonctionnelle provoquant un syndrome de négligence spatiale unilatérale en cas de lésion,
- la cartographie des zones fonctionnelles du cerveau, plus particulièrement celles concernant le domaine de la négligence spatiale unilatérale.

L'invention offre ainsi, aux praticiens, une connaissance plus précise des zones fonctionnelles du cerveau d'un patient, par exemple avant d'intervenir sur celui-ci.

La négligence spatiale unilatérale désigne une difficulté à détecter, à identifier ou à s'orienter vers des stimuli situés dans la moitié de l'espace controlatéral à l'hémisphère lésé. Concrètement ce sont des personnes ayant des comportements très handicapants qui peuvent sembler étranges comme par exemple, négliger tous les éléments extérieurs présentés sur la gauche, ne manger que la moitié de son assiette, se cogner l'épaule gauche lors du passage d'une porte, ne se maquiller ou ne se raser que la moitié du visage, comme si l'espace de gauche n'existait plus.

La négligence spatiale unilatérale est causée par une lésion cérébrale.

La présence d'une tumeur cérébrale chez un patient peut parfois nécessiter un acte chirurgical d'ablation. Dans ce cas précis il est essentiel de retirer la partie cérébrale tumorale sans endommager les zones périphériques fonctionnelles et ainsi éviter un syndrome de négligence spatiale unilatérale post-opératoire chez le patient.

Actuellement, une zone fonctionnelle du cerveau est détectée de la manière suivante : le patient est réveillé pendant l'opération. Des batteries d'évaluation de la négligence spatiale unilatérale lui sont proposées pendant que des zones de son cerveau sont perturbées par une stimulation électrique.

L'une des batteries les plus connues est la Batterie d'Evaluation de la Négligence (BEN), composée de plusieurs épreuves papier-crayon. Parmi ces tests, on retrouve des tests classiques comme les cloches consistant à entourer le plus de cloches présentes sur une feuille parmi d'autres items distracteurs, le barrage de traits consistant à barrer tous les traits présentés sur une feuille sans items distracteurs. Dans tous les cas, les patients négligents "négligent" les items cibles présentés dans leur hémi espace gauche.

Cependant, tel qu'indiqué plus haut ces tests sont réalisés à l'aide d'un crayon et de papier, et il n'existe actuellement aucun système permettant de réaliser ces tests de manière plus rapide et plus efficace, d'une part, pour le chirurgien et d'autre part pour le patient, surtout si on prend en compte le fait que ces tests sont réalisés lors d'une opération à crâne ouvert.

De plus, ces tests ne permettent pas une détection automatisée et précise de la zone fonctionnelle. Une telle détection présente une importance capitale car elle permet de réaliser l'ablation de la presque totalité d'une tumeur sans handicaper le patient par la destruction d'une partie fonctionnelle du cerveau.

II est aussi très important de réaliser une telle détection en un minimum de temps et de pouvoir fournir un outil permettant d'accompagner le chirurgien pendant l'opération ou de préparer celle-ci.

Un but de l'invention est de pallier ces inconvénients.

Un autre but de l'invention est de proposer un système automatisé de détection d'une zone fonctionnelle du cerveau d'une personne entrainant chez ladite personne une négligence spatiale unilatérale en cas de lésion.

Enfin, un autre but de l'invention est de fournir un système réalisant une détection plus efficace d'une zone fonctionnelle du cerveau d'une personne, pratique à utiliser lors d'une opération et présentant moins de danger pour le patient.

On connaît les publications suivantes.

Dans *"*New concepts in surgery of WHO grade II gliomas: functional brain mapping, connectionism and plasticity - a review"' (Hugues Duffau, Journal of neuro-oncology, kluwer academic publishers, bo, vol. 79, no. 1,77-115, 11 avril 2006) et *"*Direct evidence fora parietal-frontal pathway subserving spatial awareness in humans" (De Schotten Michel Thiebaut et al, science, vol. 309, no. 5744, 2226-2228, 30 septembre 2005) il est décrit la stimulation de zones du cerveau lors d'une tâche de bissection de ligne manuelle durant une intervention chirurgicale éveillée, afin d'étudier l'apparition d'une négligence spatiale latérale pour certaines zones stimulées.

Dans *"*Touch-screen system for assessing visuo-motor exploratory skills in neuropsychological disorders of spatial cognition" (Rabuffetti M et al., medical and biological engineering and computing, springer, heildelberg, de, vol. 40, no. 6, 675-686, 1 novembre 2002) divulgue un test assisté par ordinateur doté d'un écran tactile pour évaluer l'exploration visuomotrice de l'espace extra-personnel.

WO 2007/010242 décrit l'utilisation d'un agoniste noradrénergique dans le traitement d'un patient souffrant d'un trouble cognitif provenant d'un accident cérébral acquis, ledit trouble cognitif comprenant, par exemple, une hémi-négligence spatiale.

L'invention permet d'atteindre les buts précités par un système de détection d'une zone fonctionnelle du cerveau d'une personne entrainant chez ladite personne une négligence spatiale unilatérale en cas de lésion, ledit système étant défini dans la revendication 1. En particulier, il comprend :
- au moins une électrode adaptée pour induire un stimulus en une position du cerveau de ladite personne,
- des moyens d'affichage d'une forme géométrique suite audit/pendant ledit stimulus,
- des moyens de sélection permettant à ladite personne de pointer, selon une consigne prédéterminée, une position par rapport à ladite forme géométrique,
- des moyens de détection de la position pointée,
- des moyens de comparaison de ladite position pointée à une position calculée selon ladite consigne, et
- des moyens d'émission d'un signal en fonction de ladite comparaison.

Le système selon l'invention permet de réaliser une détection automatisée d'une zone fonctionnelle du cerveau.

La détection d'une zone fonctionnelle par le système selon l'invention est plus pratique et plus sûr qu'une détection de l'état de la technique. En effet, avec le système selon l'invention l'opérateur peut porter son attention entièrement sur le cerveau du patient et sur chacune des positions de l'électrode dans le cerveau car un signal préviendra l'opérateur des résultats de l'application de la consigne par la personne concernée tout au long du test.

Par ailleurs, la détection d'une zone fonctionnelle du cerveau par le système selon l'invention est plus rapide, car les moyens utilisés sont des moyens électroniques et/ou informatiques permettant à la personne et à l'opérateur de réaliser la détection sans interruption.

De plus, le système de détection d'une zone fonctionnelle selon l'invention peut être utilisé directement en salle opératoire avec un accès aux résultats immédiat, ou préalablement à une opération sur le patient.

Dans un mode de réalisation particulier, les moyens de détection de la position pointée peuvent comprendre un écran tactile, ledit écran tactile réalisant aussi l'affichage de ladite forme géométrique.

L'écran tactile peut être utilisé avec un pointeur tel qu'un stylet ou directement avec le doigt.

Ainsi, les moyens d'affichage et les moyens de sélection comprennent un écran unique.

Dans une version particulièrement avantageuse, les moyens d'émission d'un signal sont adaptés à émettre un signal différent pour chacun des cas suivants :
- la distance entre la position pointée et la position calculée est inférieure à une distance prédéterminée,
- la distance entre la position pointée et la position calculée est supérieure à ladite distance prédéterminée mais inférieur au double de ladite distance,
- la distance entre la position pointée et la position calculée est supérieure au double de ladite distance prédéterminée.

Ainsi, en fonction du signal émis, l'opérateur peut savoir directement si la position stimulée se trouve dans une zone fonctionnelle du cerveau ou non.

Le système comprend en outre des moyens de détermination de la position du stimulus. Ces moyens peuvent comprendre une caméra filmant la scène et donc la position de chacun des stimuli.

Le système selon l'invention peut en outre comprendre des moyens de mémorisation du résultat de la comparaison en association avec la position du stimulus, pour une pluralité de positions différentes.

Les moyens de mémorisation peuvent être embarqués ou se trouver dans une base de données distante. Dans ce cas, le système peut en outre comprendre des moyens de connexion à la base distante, ces moyens de connexion pouvant comprendre des moyens de connexion sans fil et/ou des moyens de connexion filaire au travers d'un réseau existant par exemple.

Avantageusement, le système selon l'invention comprend en outre des moyens de cartographie de la zone fonctionnelle en fonction de la position de chacun des stimuli et du résultat de la comparaison pour ladite position, à partir d'une carte du cerveau de la personne, obtenue par des moyens d'imagerie médicale connue de l'homme du métier, telle que des moyens d'imagerie par résonnance magnétique (IRM). Ainsi, une cartographie en 2 ou 3 dimensions de la zone fonctionnelle dans le cerveau peut être réalisée.

La carte réalisée peut être utilisée lors de l'opération chirurgicale sur le cerveau ou avant l'opération pour préparer l'ablation de la tumeur cérébrale.

Une telle carte du cerveau faisant apparaître la zone fonctionnelle permet de mieux visualiser la zone fonctionnelle dans le cerveau.

Les moyens de cartographie peuvent comprendre un ou plusieurs programmes informatiques.

Selon une version particulièrement avantageuse, les moyens d'émission d'un signal comprennent des moyens d'émission d'un signal sonore. Le signal sonore peut être de fréquence, de durée ou d'itérations différentes en fonction du résultat de la comparaison.

Un signal sonore présente l'avantage pour l'opérateur de ne pas avoir à détourner son attention visuelle du cerveau de la personne pour connaître le résultat de la comparaison, ce qui diminue le risque de mauvaises manipulations de la part de l'opérateur qui pourraient avoir des conséquences importantes sur la santé de la personne.

Les moyens d'émission d'un signal peuvent comprendre des moyens d'émission d'un signal visuel.

Un tel signal visuel peut en outre être utilisé en complément d'un signal sonore.

D'autres avantages et caractéristiques de l'invention apparaîtront à l'examen de la description détaillée d'un mode de mise en oeuvre nullement limitatif, et des dessins annexés sur lesquels :
- la figure 1 est une représentation schématique d'un système selon l'invention ; et
- la figure 2 est un exemple d'un test utilisé par le système de la figure 1 pour la détermination d'une zone fonctionnelle du cerveau d'une personne souffrant de négligence spatiale unilatérale.

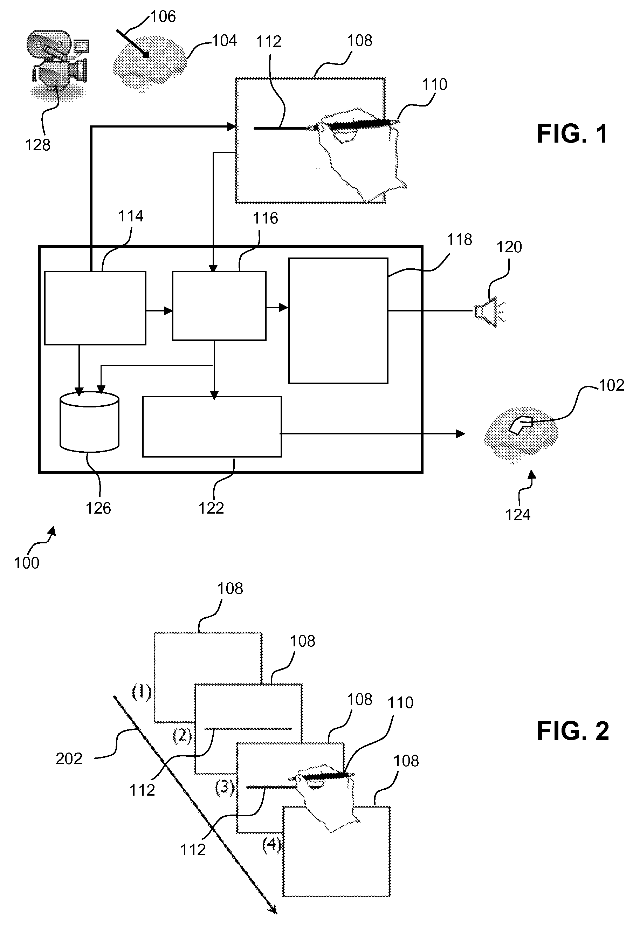

La figure 1 est une représentation schématique d'un système 100 de détection d'une zone fonctionnelle 102 du cerveau 104 d'un patient dont la lésion provoque un syndrome de négligence spatiale unilatérale.

Le système 100 comprend :
- au moins une électrode 106 adaptée pour induire un stimulus en une position du cerveau 104 du patient. Chaque stimulus est un signal électrique de très faible tension et de quelques mA sur le cerveau 104 du patient ;
- un écran tactile 108 sur lequel sont affichées une ou plusieurs formes géométrique pendant ou juste après le stimulus ;
- un stylet 110 permettant au patient de pointer une position sur l'écran tactile 108 conformément à une consigne prédéterminée. La position à pointer est demandée par rapport à la forme géométrique. Dans l'exemple représenté sur la figure 1 la forme géométrique est un segment 112 et la consigne est de pointer le milieu du segment 112 avec le stylet 110 ;
- un module 114 de génération de la forme géométrique sur l'écran d'affichage 108. Ce module 114 de génération de forme détermine la position d'affichage du segment 114 sur l'écran d'affichage 108, ainsi que la consigne, notamment la position qui doit être pointée par le patient, à savoir le milieu du segment 112.

La position pointée par le patient avec le stylet 110 est détectée par l'écran tactile 108 et communiqué à un module de comparaison 116. Ce module de comparaison 116 reçoit, de l'écran tactile 108, la position pointée par le patient et, du module de génération de stimuli 114, la position exacte que le patient devrait pointer en fonction de la consigne. Le module de comparaison 116 compare les deux positions et détermine, en fonction d'un ou plusieurs seuils prédéterminés, le niveau d'exactitude de la réponse donnée par le patient.

Le niveau d'exactitude est communiqué à un module 118 de génération d'un signal sonore. Ce module 118 génère un signal sonore différent en fonction du niveau d'exactitude. Le signal sonore associé au niveau d'exactitude communiqué par le module de comparaison 116 est émis par un haut parleur 120 relié au module de génération d'un signal sonore 118.

Le niveau d'exactitude ainsi que la position de l'électrode 106 sont également communiqués à un module de cartographie 122. En fonction du niveau d'exactitude pour une multitude de position de stimuli sur le cerveau 104, le module de cartographie 122 génère une carte 124 en trois dimensions du cerveau 104 sur lequel est représentée la zone fonctionnelle 102 du cerveau 104, ici la zone qui lorsqu'elle est stimulée induit une négligence spatiale unilatérale.

La génération de la carte 124 en trois dimensions du cerveau 104 par le module de génération 122 peut être réalisée soit en temps réel lors du test du patient avec les stimuli soit de manière décalée après le test.

La carte générée 124 peut être affichée sur un écran d'affichage non représenté sur la figure 1.

Tout ou partie des données ainsi que la carte d'affichage peuvent être stockée dans une base de données 126.

Le système selon l'invention peut en outre comprendre des moyens de conversion de la carte 124 dans un langage prédéterminé pour que la carte 124 puisse être utilisée par un appareil numérique d'aide à la chirurgie lors de l'ablation de la zone de la tumeur cérébrale.

Le système peut en outre comprendre une camera 128 filmant les actions du chirurgien opérateur et enregistrant les signaux sonore émis. Les données enregistrées par la caméra 128 peuvent être utilisées par le module de génération 122 pour la génération de la carte 124 du cerveau en trois dimensions et faisant apparaître la zone fonctionnelle du cerveau du patient.

La figure 2 est une représentation d'un exemple de test d'évaluation de la négligence spatiale unilatérale, utilisé par le système de la figure 1 pour déterminer une zone fonctionnelle du cerveau.

Il s'agit d'un test cognitif informatisé de bissection de segment utilisant l'écran tactile 108. Un signal sonore aigu accompagne l'apparition d'une ligne horizontale de 20 cm. La consigne donnée au patient est de pointer le centre du segment affiché sur l'écran tactile 108. Le sujet doit donc marquer à l'aide d'un stylet le centre du segment.

Un signal sonore grave d'erreur indique si le sujet se trouve à plus de 6,5 mm (distance prédéterminée) du centre (simple bip) ou plus de 13mm du centre (double bip). Il n'y a pas de signal sonore grave si le sujet marque la ligne précisément au centre (moins de 6,5mm d'erreur). Les seuils de 6,5mm (distance prédéterminée) et de 13mm (double de la distance prédéterminée) ne sont pas arbitraires mais correspondent à des seuils définis au préalable dans la littérature comme statistiquement anormaux.

Ce test peut notamment être utilisé en milieu pré-opératoire, pour ne pas handicaper le patient lors d'une intervention neurochirurgicale.

Dans un mode de réalisation particulier, un exemple de système testé comprend un ordinateur portable avec écran tactile et stylet de réponse, avec système d'exploitation Windows XP.

Un programme informatique réalisant les fonctions décrites plus haut tournent en boucle jusqu'à l'appui sur la touche « échap ».

Une pluralité de stimuli est affichée progressivement. L'axe 202 sur la figure 2 symbolise le temps et les numéros entre parenthèse sur la figure 2 symbolisent l'ordre des stimuli affichés sur l'écran tactile 108 au fur et à mesure du test. La durée d'affichage des différents stimuli est différente. Selon un exemple particulier :
- le stimulus (1) comporte deux scènes une première de 1000ms, puis un signal sonore, puis une deuxième scène de 500ms suivi d'un signal sonore,
- le stimulus (2) une scène de 5000ms,
- le stimulus (3) aucune scène n'est affichée, et
- le stimulus (4) un signal sonore suivi d'une scène de 500ms.

A chaque essai le programme informatique enregistre la réponse dans un fichier texte et émet un signal sonore suivant la réponse.

La réponse enregistrée est constituée du temps de réponse à partir de l'apparition de la ligne, la position en pixels du point marqué par le sujet, la distance en millimètres de ce point par rapport au centre de la ligne, le pourcentage de déviation et le coté de la déviation.

Si le sujet ne répond pas au bout de 5 secondes un signal sonore d'erreur est émis et le programme enregistre dans le fichier de réponse qu'il n'y a pas eu de réponse à cet essai.

Bien sûr, l'invention n'est pas limitée aux exemples qui viennent d'être décrits et de nombreux aménagements peuvent être apportés à ces exemples sans sortir du cadre de l'invention.

## Revendications

1. Système de détection d'une zone fonctionnelle (102) du cerveau (104) d'une personne entrainant chez ladite personne une négligence spatiale unilatérale en cas de lésion, ledit système comprenant :
- au moins une électrode (106) adaptée pour induire un stimulus en une position du cerveau (104) de ladite personne,
- des moyens d'affichage (108) d'une forme géométrique (112) suite audit stimulus,
- des moyens de sélection (110) permettant à ladite personne de pointer, selon une consigne prédéterminée, une position par rapport à ladite forme géométrique (112),
**caractérisé en ce qu'**il comprend en outre :
- des moyens (114) de détermination de la position du stimulus,
- des moyens de détection (108) de la position pointée,
- des moyens (116) de comparaison de ladite position pointée à une position calculée selon ladite consigne,
- des moyens d'émission (118,120) d'un signal en fonction de ladite comparaison,
- des moyens de mémorisation (126) du résultat de la comparaison en association avec une position du stimulus, pour une pluralité de positions différentes,
- des moyens de cartographie (122) de la zone fonctionnelle (102) entrainant chez ladite personne une négligence spatiale unilatérale, en fonction de la position de chacun des stimuli et du résultat de la comparaison pour chaque position de stimuli, à partir d'une carte du cerveau de ladite personne obtenue par des moyens d'imagerie médicale.

2. Système selon la revendication 1, **caractérisé en ce que** les moyens de détection de la position pointée comprennent un écran tactile (108), ledit écran tactile (108) réalisant aussi l'affichage de ladite forme géométrique (112).

3. Système selon l'une quelconque des revendications précédentes, **caractérisé en ce que** les moyens d'émission (118,120) d'un signal sont adaptés à émettre un signal différent pour chacun des cas suivants :
- la distance entre la position pointée et la position calculée est inférieure à une distance prédéterminée,
- la distance entre la position pointée et la position calculée est supérieure à ladite distance prédéterminée mais inférieur au double de ladite distance,
- la distance entre la position pointée et la position calculée est supérieure au double de ladite distance prédéterminée.

4. Système selon l'une quelconque des revendications précédentes, **caractérisé en ce que** les moyens d'émission d'un signal comprennent des moyens (118,120) d'émission d'un signal sonore.

5. Système selon l'une quelconque des revendications précédentes, **caractérisé en ce que** les moyens d'émission d'un signal comprennent des moyens d'émission d'un signal visuel.

6. Système selon l'une quelconque des revendications précédentes, **caractérisé en ce que** la carte du cerveau de ladite personne est une carte en trois dimensions.

## Patentansprüche

1. System zur Erkennung eines Funktionsbereichs (102) des Gehirns (104) einer Person, der im Falle einer Läsion, bei dieser Person einen visuellen Neglect verursacht, wobei das System Folgendes umfasst:
- mindestens eine Elektrode (106), die dazu ausgebildet ist, in einer Position des Gehirns (104) der Person einen Reiz auszulösen,
- Anzeigemittel (108) zur Anzeige einer geometrischen Form (112) in Folge des Reizes,
- Auswahlmittel (110), die es der Person erlauben, gemäß einer vorbestimmten Anweisung, auf eine Stelle im Verhältnis zur geometrischen Form (112) zu zeigen, **dadurch gekennzeichnet**, das es außerdem Folgendes umfasst:
- Bestimmungsmittel (114) zur Bestimmung der Position des Reizes,
- Erkennungsmittel (108) zur Erkennung der gezeigten Position,
- Vergleichsmittel (116) zum Vergleich der gezeigten Position mit einer gemäß der vorbestimmten Anweisung errechneten Position,
- Ausgabemittel (118, 120) zur Ausgabe eines vom Vergleich abhängigen Signals,
- Speichermittel (126) zum Speichern des Ergebnisses des Vergleichs zusammen mit einer Position des Reizes für eine Vielzahl von verschiedenen Positionen,
- Mittel (122) zum Kartographieren des Funktionsbereichs (102), welcher bei der Person einen visuellen Neglect verursacht, anhand der Position jedes Reizes und des Ergebnisses des Vergleichs für jede Position des Reizes, auf der Grundlage einer durch medizinische Bildgebungsmittel erstellen Karte des Gehirns der Person.

2. System nach Anspruch 1, **dadurch gekennzeichnet, dass** die Erkennungsmittel zur Erkennung der gezeigten Position ein Touchscreen (108) umfassen, wobei dieses Touchscreen (108) auch der Anzeige der geometrischen Form (112) dient.

3. System nach einem der vorhergehenden Ansprüche, **dadurch gekennzeichnet, dass** die Ausgabemittel (118, 120) eines Signals dazu eingerichtet sind, in jedem der folgenden Fälle ein unterschiedliches Signal auszugeben:
- der Abstand zwischen der gezeigten Position und der errechneten Position ist kleiner als ein vorbestimmter Abstand,
- der Abstand zwischen der gezeigten Position und der errechneten Position ist grösser als der vorbestimmte Abstand aber kleiner als das Zweifache des Abstands,
- der Abstand zwischen der gezeigten Position und der errechneten Position ist grösser als das Zweifache des vorbestimmten Abstands.

4. System nach einem der vorhergehenden Ansprüche, **dadurch gekennzeichnet, dass** die Ausgabemittel zur Ausgabe eines Signals Mittel (118, 120) zur Ausgabe eines akustischen Signals umfassen.

5. System nach einem der vorhergehenden Ansprüche, **dadurch gekennzeichnet, dass** die Ausgabemittel zur Ausgabe eines Signals Mittel zur Ausgabe eines optischen Signals umfassen.

6. System nach einem der vorhergehenden Ansprüche, **dadurch gekennzeichnet, dass** die Karte des Gehirns der Person eine dreidimensionale Karte ist.

## Claims

1. A system for detecting a functional area (102) of a person's brain (104) leading in said person to hemispatial neglect in case of lesion, said system comprising:
- at least one electrode (106) adapted to induce a stimulus in a position of the brain (104) of said person,
- means (108) for displaying a geometric shape (112) further to said stimulus,
- selection means (110) enabling said person to point, according to a predetermined instruction, to a position relative to said geometric shape (112), **characterized in that** it further comprises:
- means (114) for determining the position of the stimulus,
- means for detecting (108) the position pointed to,
- means (116) for comparing said pointed to position to a position computed according to said instruction, and
- means (118,120) for emitting a signal on the basis of said comparison,
- means (126) for storing the result of the comparison in association with a position of the stimulus, for a plurality of different positions,
- means (122) for mapping the functional area (102) on the basis of the position of each of the stimuli and the result of the comparison for each position of the stimuli, on the basis of a map of the brain of said person obtained by means for medical imaging.

2. A system according to claim 1, **characterized in that** the means for detecting the position pointed to comprise a touch screen (108), said touch screen (108) also displaying said geometric shape (112)

3. A system according to any one of the preceding claims, **characterized in that** the means (118,120) for emitting a signal are adapted to emit a different signal for each of the following cases:
- the distance between the position pointed to and the computed position is less than a predetermined distance,
- the distance between the position pointed to and the computed position is greater than said predetermined distance but less than twice said distance,
- the distance between the position pointed to and the computed position is greater than twice said predetermined distance.

4. A system according to any one of the preceding claims, **characterized in that** the means for emitting a signal comprise means (118,120) for emitting an acoustic signal.

5. A system according to any one of the preceding claims, **characterized in that** the means for emitting a signal comprise means for emitting a visual signal.

6. A system according to anyone of the preceding claims, **characterized in that** the map of the brain of said person is a map in three dimensions.
